Europäisches Patentamt

European Patent Office

Office européen des brevets

(19)

(11) **EP 0 811 578 A2**

(12) **EUROPÄISCHE PATENTANMELDUNG**

(43) Veröffentlichungstag:
    **10.12.1997 Patentblatt 1997/50**

(51) Int. Cl.[6]: **C02F 1/32**, A61L 2/10,
    B01J 19/12, G01J 1/42

(21) Anmeldenummer: 97109179.8

(22) Anmeldetag: **06.06.1997**

(84) Benannte Vertragsstaaten:
    **AT BE CH DE DK ES FI FR GB GR IE IT LI LU MC
    NL PT SE**

(30) Priorität: **06.06.1996 DE 29609998 U**

(71) Anmelder: **RICOH KYOSAN INC.
    Tokyo 104 (JP)**

(72) Erfinder: **Rehman, Zillur
    79312 Emmendingen (DE)**

(74) Vertreter:
    **Blum, Rudolf Emil Ernst et al
    c/o E. Blum & Co
    Patentanwälte
    Vorderberg 11
    8044 Zürich (CH)**

(54) **Vorrichtung zur Entkeimung eines Fluides**

(57)     Eine erste Zufuhrleitung (19) für eine gesteuerte Menge eines Desinfektionsmittels führt in die Zufuhrleitung (12) des zu behandelnden Fluids. In einem nachfolgenden Mischreaktor (23) wird dem Fluid-Desinfektionsmittel-Gemisch eine gesteuerte Menge $CO_2$ zugeführt. Das gesamte Gemisch wird in die UV-Anlage (1) eingeführt. Durch eine weitere Zufuhrleitung (20) wird dem aus der UV-Anlage (1) austretendem behandeltem Fluid gesteuert weiteres Desinfektionsmittel zugeführt werden. Damit weist die Entkeimungsvorrichtung eine äusserst grosse Flexibilität auf und der Verbrauch von Desinfektionsmittel wird reduziert.

Fig. 1

EP 0 811 578 A2

## Beschreibung

Die vorliegende Erfindung betrifft eine Vorrichtung zur Entkeimung eines Fluides, mit einer UV-Anlage einschliesslich UV-Lampten, einer UV-Intensitätssteuereinheit, einer UV-Intensitätsmessonde und einer mechanischen UV-Lampen-Reinigungseinrichtung, welche UV-Anlage eine Fluidzulaufleitung und eine Fluidauslaufleitung aufweist.

Für die Entkeimung verschiedener Medien, wie Luft, Festkörper und Flüssigkeiten, werden chemische Desinfektion, Filtration, thermische Behandlung sowie Strahlentechnologien verwendet.

In den meisten Fällen wird die chemische Desinfektion verwendet. Wasser und Prozesswasser werden mittels Desinfektionschemikalien, wie z.B. Chlorbleichlauge (NaOCl), Chlordioxid, Peressigsäure, etc. entkeimt. Festkörper wie Tanks, Rohrleitungen etc. werden entkeimt, indem Wasser mit einem Desinfektionsmittel versetzt wird und die betreffenden Festkörper ausgespült bzw. durchgespült werden.

Luft wird üblicherweise mittels der UV-Technologie entkeimt. Weiter gibt es viele Flüssigkeiten, Festkörper und allgemeine Gase, die aus gesetzlichen und aus produktionstechnischen Gründen z.B. eine chemische Desinfektion nicht zulassen. Beispielsweise wird Mineralwasser durch Filtration, Verschlüsse für Getränkeflaschen thermisch oder mittels UV-Strahlen entkeimt. Bekannt ist auch die UV-Behandlung von Trinkwasser.

Die chemische Desinfektion weist verschiedene Nachteile auf. Bei Ueber- oder Unterdosierung des Desinfektionsmittels können Korrosionen entstehen, es können gefährliche Zwischenverbindungen gebildet werden, es kann zur Abwasser- oder Abluftbelastung führen. Weiter wird für die Spülung ein erhöhter Wasserbedarf notwendig sein, die Produktequalität wird gefährdet, Mikroorganismen werden resistenter und weiter führt die chemische Desinfektion zu einem ständigen Wechsel der Chemikalien, bzw. zu Konzentrationserhöhungen.

Der Entkeimung mittels UV-Technologien sind ebenfalls Grenzen gesetzt. Die UV-Entkeimung hat keine Depotwirkung, d.h. es besteht die Gefahr der Reinfektion (nach der UV-Ablage). Ablagerungen z.B. von Kalk, Fasern, etc. auf dem UV-Strahler vermindern die Entkeimungsleitung. UV-Anlagen bedingen oft hohe Investitionskosten und sind weiter zur Entkeimung von Festkörpern wie Rohrleitungen und Behälter nicht einsetzbar. Weist das betreffende Medium bzw. das Fluid eine hohe UV-Absorption auf, ist die UV-Entkeimung nur bedingt einsetzbar. Schliesslich ist die Entkeimungssicherheit nur dann gegeben, wenn alle Parameter wie Durchfluss, Eingangskeimzahlen und Zusammensetzung des Mediums nicht ändern. Im Falle einer Veränderung wird die UV-Ablage unbrauchbar.

Ziel der Erfindung ist eine Vorrichtung zur Entkeimung eines Fluides zu zeigen, welche die Vorteile beider Entkeimungsmethoden in sich vereinigt, eine erhöhte Entkeimungswirkung mit minimaler Desinfektionsmittel-konzentration aufweist und eine erhöhte Betriebssicherheit hat.

Die erfindungsgemässe Vorrichtung ist gekennzeichnet durch eine erste Desinfektionsmittel-Zufuhreinrichtung, die eine in die Fluidzufuhrleitung der UV-Anlage mündende erste Desinfektionsmittel-Zufuhrleitung aufweist, eine zweite Behandlungsmittel-Zufuhreinrichtung, die eine in die Fluidauslassleitung der UV-Ablage mündende zweite Behandlungsmittel-Zufuhrleitung aufweist, durch eine $CO_2$-Zufuhreinrichtung mit einer $CO_2$-Zufuhrleitung, die in einen an einer Stelle zwischen der ersten Desinfektionsmittel-Zufuhrleitung und der UV-Ablage in der Fluidzulaufleitung angeordneten Mischreaktor mündet, und durch eine Sensorenanlage, die dazu dient, den Zustand des Fluides in der Fluidzulaufleitung oder der Fluidauslaufleitung abzutasten.

Vorteilhafte Ausführungen ergeben sich aus den abhängigen Ansprüchen.

Die durch die Erfindung erreichten Vorteile sind, dass die gesamte Vorrichtung somit eine höhere Entkeimungssicherheit und Flexibilität bietet, als dies mit nur UV oder nur Chemie allein möglich ist. Ferner wird der Verbrauch von Desinfektionsmitteln erheblich reduziert und damit die Korrosion in Anlageteilen, die Abwasserbelastung und auch Veränderungen der Qualität von entsprechenden Produkten.

Nachfolgend wird der Erfindungsgegenstand anhand der Zeichnungen beispielsweise näher erläutert. Es zeigt:

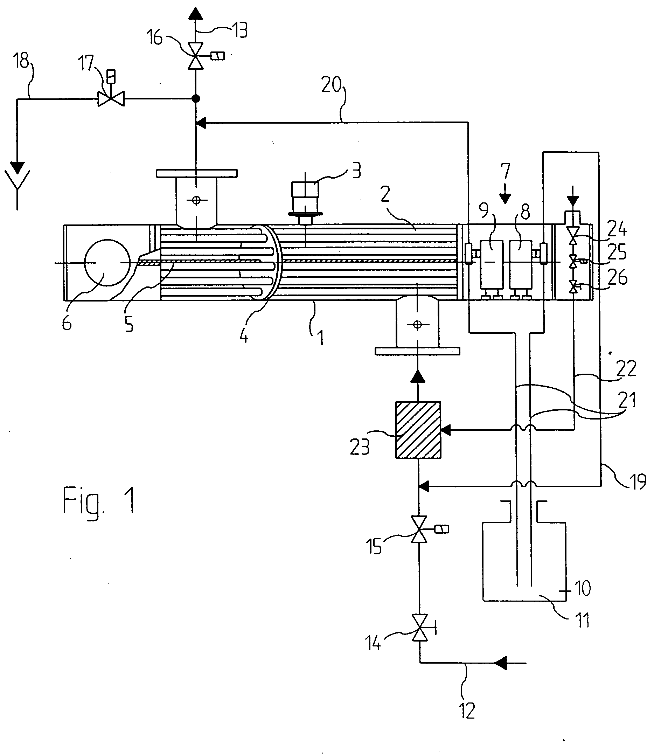

Figur 1 schematisch eine Entkeimungsvorrichtung, und
Figur 2 ein Diagramm zur Erläuterung der Betriebsweise der Entkeimungsvorrichtung.

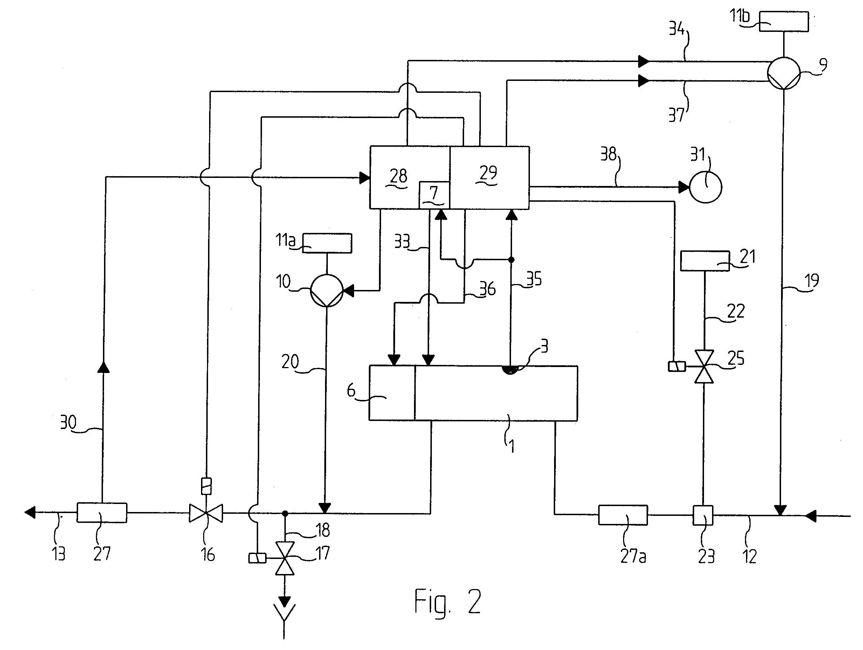

Die Vorrichtung weist eine UV-Anlage 1 auf, in deren Kammer die in Quarzröhren angeordneten UV-Lampen 2 angeordnet sind. Weiter ist eine Intensitätsmessonde 3 zur Messung der UV-Intensität vorhanden. Um die UV-Lampen bzw. Röhren von Ablagerungen z.B. Kalk zu reinigen, enthält die UV-Anlage eine mechanische Reinigungseinrichtung. Diese Reinigungsvorrichtung besteht hauptsächlich aus einem Schaber 4 in Form einer gelochten Teflonscheibe, die von den UV-Lampen mit den diese umgebenden Quarzröhren durchsetzt ist. Dieser Schaber 4 sitzt auf einer Spindel 5. Die Spindel 5 wird von einem Motor 6 der Spindelantriebsvorrichtung angetrieben. Die UV-Leistung ist durch eine Regeleinrichtung 7 in drei Stufen regelbar. Diese Regeleinrichtung ist schematisch in Fig. 2 angedeutet. In einer an die die UV-Lampen enthaltende anschliessenden Kammer 8 sind eine erste 9 und eine zweite Dosierpumpe 10 angeordnet. Saugseitig stehen diese Dosierpumpen mit einem Desinfektionsmittelbehälter 11 in Verbindung, wobei wie in Fig. 2 dargestellt ist, jede Dosierpumpe 9 bzw. 10 auch mit einem eigene Desinfektions- oder Behandlungsmittelbehälter 11a bzw. 11b in Verbindung stehen kann.

Die Zulaufleitung des zu entkeimenden Fluides ist allgemein mit der Bezugsziffer 12 bezeichnet, und die Auslaufleitung ist mit der Bezugsziffer 13 bezeichnet. In der Zulaufleitung 12 sind ein Regelventil 14 und ein Magnetventil 15 angeordnet. In der Auslaufleitung 13 ist ein weiteres Magnetventil 16 angeordnet. Von der Auslaufleitung 13 zweigt weiter eine mit einem Magnetventil 17 ausgerüstete Kanalablassleitung 18 ab. Von der ersten Dosierpumpe 9 führt eine Desinfektionsmittel-Zufuhrleitung 19 in die Zulaufleitung 12. Von der zweiten Dosierpumpe 10 führt eine weitere Desinfektionsmittel-Zufuhrleitung 20 in die Auslaufleitung 13.

Durch diese Zufuhrleitung 20 kann nochmals ein Desinfektionsmittel und/oder können andere Zugaben, z.B. Vitamine zugeführt werden, wobei gewisse Vitamin-Zugaben in sich zusätzlich eine Desinfektion bewirken können.

Von einer $CO_2$-Quelle 21 führt eine $CO_2$-Zufuhrleitung 22 zu einem Fluid/$CO_2$-Mischreaktor 23, der in der Zulaufleitung 12 angeordnet ist. In der $CO_2$-Zufuhrleitung 22 sind ein Druckminderer 24, ein Magnetventil 25 und ein Regelventil 26 angeordnet.

Mittels einer Sensorenanlage 27 (Fig. 2) wird der Zustand des entkeimten Fluides ermittelt, z.B. Durchfluss, Temperatur, Trübung, Keimzahlen, Viskosität, Redoxpotential, pH-Wert, etc. Alternativ oder zusätzlich zur Sensorenanlage 27 kann auch eine Sensorenanlage 27a in der Zufuhrleitung 12 angeordnet sein.

Aus der obigen Beschreibung der Entkeimungsvorrichtung geht allgemein hervor, dass vor und nach der UV-Anlage 1 dem zu entkeimenden Fluid ein Desinfektionsmittel zugegeben wird, bzw. werden kann.

Durch die Desinfektionsmittel-Zufuhrleitung 19 wird dem zu entkeimenden Fluid ein Desinfektionsmittel zugegeben, was durch die in der UV-Anlage nachfolgende Wechselwirkung von UV mit der chemischen Desinfektion eine verstärkte Desinfektionsleistung zur Folge hat. Es entstehen Hydroxid-Radikale, die eine wesentlich höhere Entkeimungswirkung als nur die chemische Desinfektion haben, z.B.

$$O3 + UV \rightarrow O_2 + O\dot{}$$

$$O\dot{} + HOH \rightarrow 2\,OH\dot{}$$

$$H_2O_2 + UV \rightarrow HOH + O\dot{}$$

$$O\dot{} + HOH \rightarrow 2\,OH\dot{}$$

Somit lässt sich die Desinfektionsmittelkonzentration reduzieren und die Entkeimungswirkung erhöhen. Grundsätzlich wird das mit nur wenig Desinfektionsmittel angereicherte Fluid in der UV-Anlage entkeimt. Die Desinfektionsmittel, und grundsätzlich nach der UV-Anlage zugegebene Desinfektionsmittel dienen für die Absicherung des Transportes des Fluides, d.h. für die Entkeimung der Transportleitungen, um eine Reinfektion nach der UV-Anlage zu vermeiden.

Nachfolgend wird der Betrieb der neuerungsgemässen Vorrichtung insbesondere anhand der Fig. 2 erläutert. Die Steuerung des Betriebes erfolgt durch ein Steuergerät mit Mikroprozessoren, das grundsätzlich als aus einer ersten Steuereinrichtung 28 und einer zweiten Steuereinrichtung 29 aufgebaut betrachtet werden kann, wobei zur Verdeutlichung die UV-Leistungsregeleinrichtung zusätzlich als Teil 7 der ersten Steuereinrichtung 28 illustriert ist. Die Sensorenanlage 27 ist über eine Signalübertragungsleitung 30 mit der ersten Steuereinrichtung 28 verbunden. Die durch die einzelnen Sensoren der Sensorenanlage 27 ermittelten Messwerte, z.B. Durchfluss, Temperatur, Trübung, Keimzahlen, pH-Wert etc. werden der ersten Steuereinrichtung 28 zugeführt und mit gespeicherten Sollwerten verglichen. Im Falle des Vorhandenseins einer Sensorenanlage 27a in der Zulaufleitung 12 sind offensichtlich entsprechende Signalübertragungsleitungen vorhanden, die aus Gründen der Uebersichtlichkeit nicht eingezeichnet sind.

Ausgangsseitig ist die erste Steuereinrichtung 28 über eine Steuerleitung 32 mit der zweiten Dosierpumpe 10, eine Steuerleistung 33 mit der UV-Regeleinrichtung 7, und einer Steuerleitung 34 mit der ersten Dosierpumpe 9 verbunden.

Wenn nun die Messwerte in dem Sinn den Sollwerten nicht entsprechen, dass sie eine ungenügende Entkeimung anzeigen, wird vorerst von der ersten Steuereinrichtung 28 bzw. der UV-Regeleinrichtung 7 die UV-Leistung höher geschaltet.

Die Intensitätsmessonde 3 ist über die Signalleitung 35 sowohl mit der ersten Steuereinrichtung 28 als auch mit der zweiten Steuereinrichtung 29 verbunden.

Wenn nun aufgrund des von der Intensitätsmessonde 3 ausgehenden Signales in der ersten Steuereinrichtung 28 festgestellt wird, dass die Intensität einen Maximalwert erreicht hat, jedoch aufgrund der Signale der Sensorenanlage festgestellt wird, dass die Entkeimung noch nicht hoch genug ist, werden aufgrund der Signalübermittlung durch die Steuerleitungen 28 bzw. 34 die Desinfektionsmittel-Dosierpumpen 9, bzw. 10 in Betrieb gesetzt oder die Dosierung erhöht. Durch die Wechselwirkung von UV und Desinfektionsmittel wird die Entkeimung verstärkt und den bei der Sensoranlage 27 ermittelten, geänderten Parametern automatisch angepasst, so dass eine Entkeimung stets gewährleistet ist. Im Falle von anderen Zugaben, z.B. Vitamine über die Dosierpumpe 10 wird deren Förderleistung sinngemäss ebenfalls geändert.

Die Betriebssicherheit der Anlage wird insbesondere in der zweiten Steuereinrichtung 29 überwacht.

Die Intensitätsmessonde 3 steht über die Signalleitung 35 mit der zweiten Steuereinrichtung 29 in Verbindung. Von der zweiten Steuereinrichtung 29 führt eine Steuerleitung 36 sowohl zum Antriebsmotorteil 6, und weiter steht sie in Verbindung mit der UV-Intensitätsregeleinrichtung 7. Eine weitere Steuerleitung 37 führt zur ersten Dosierpumpe 9. Weiter führt eine Steuerleitung 38 zu einem Alarmgeber 31. Nach dem Einschalten der Anlage werden die UV-Lampen 2 und die zweite Desinfektions- oder Behandlungsmittel-Dosierpumpe 10, welche dem zu entkeimenden Fluid nach der UV-Anlage 1 Desinfektionsmittel zudosiert, eingeschaltet. Die Konzentration des Desinfektionsmittels wird übrigens manuell geregelt. Die Desinfektionsmittelzudosierung hilft, das Fluid vor Reinfektionen, z.B. in den Rohrleitungen zu schützen.

Eine Abnahme der Intensität wird durch die Intensitätsmessonde 3 ermittelt, deren Ausgangssignale (unter anderem) über die Signalleitung 35 der zweiten Steuereinrichtung 29 zugeführt sind. Die Ursache für die Abnahme der Intensität können Ablagerungen (z.B. Kalk) an den die UV-Lampen 2 umgebenden Quarzröhren oder UV-Licht absorbierende Verunreinigungen im Fluid sein. Sobald über die Intensitätsmessonde 3 eine Abnahme der UV-Intensität festgestellt wird, wird die mechanische Reinigung eingeschaltet und die UV-Intensität auf eine höhere Leistungsstufe geregelt. Konkret wird über die zweite Steuereinrichtung 29 der Motor 6 in Betrieb gesetzt, so dass die Spindel 5 rotiert und der Schaber 4 entlang den Quarzröhren verschoben wird, um die Ablagerungen mechanisch zu entfernen. Gleichzeitig wird die UV-Regeleinrichtung 7 auf eine höhere Leistungsstufe geregelt. Die höhere Leistungsstufe gewährleistet die notwendige UV-Energie im zu entkeimenden Fluid und das Vorhandensein von Verunreinigungen wird auf diese Weise kompensiert. Nimmt die UV-Intensität noch weiter ab, wird nach Erreichen eines festgelegten Minimalwertes der Intensität über die Steuerleitung 37 die erste Dosierpumpe 9 eingeschaltet und Desinfektionsmittel vor der UV-Anlage 1 dem zu entkeimenden Fluid zugeführt. Auf diese Weise entsteht, wie eingangs erwähnt, ein hohes Entkeimungspotential in der gesamten Anlage, das bei einer UV-Intensität von nahezu Null immer noch eine ausreichende Entkeimung gewährleistet.

Im Falle, dass sich sehr viel Kalk auf den Quarzröhren ablagert, also die Intensität sehr tief fällt, wird über die Steuerleitung 38 der Alarmgeber 31 in Betrieb gesetzt. Ist der Alarm ausgelöst worden, wird manuell die $CO_2$-Zufuhr eingeleitet und damit eine $CO_2$-Reinigung der Quarzröhren bewirkt. Das $CO_2$ löst die gebildeten Karbonate auf den Quarzröhren unter Bildung von Hydrogenkarbonaten ab. Während der $CO_2$-Reinigung bleibt die mechanische Reinigung eingeschaltet.

Die $CO_2$-Reinigung verläuft wie folgt. Das Magnetventil 25 der $CO_2$-Zufuhrleitung 22 wird geöffnet, und das Magnetventil 16 der Auslaufleitung 13 wird unmittelbar danach geschlossen. Während einer gewissen Verweilzeit kann das $CO_2$ auf die Ablagerungen einwirken, welche auch durch das fortgesetzte mechanische Reinigen von den Quarzröhren abgestreift werden. Danach wird das Magnetventil 17 geöffnet, um das Fluid mit den gelösten Verunreinigungen, Ablagerungen aus der Kammer der UV-Anlage 1 durch die Kanalablassleitung 18 abzuführen. Dieser Vorgang wird mehrere Male wiederholt, bis über die Intensitätsmessonde festgestellt wird, dass von den UV-Lampen wieder die volle Leistung abgegeben wird.

Bei sehr hohen Keimzahlen im zu entkeimenden Fluid oder bei einer Erhöhung des Durchflusses kann die UV-Leistung auf eine höhere Leistungsstufe geregelt wund die Desinfektionsmittelzudosierung vor und nach der UV-Anlage 1 eingeschaltet werden.

**Patentansprüche**

1. Vorrichtung zur Entkeimung eines Fluids, mit einer UV-Anlage (1) einschliesslich UV-Lampen (2), einer UV-Intensitätssteuereinheit (7), einer UV-Intensitätsmessonde (3) und einer mechanischen UV-Lampen-Reinigungseinrichtung, welche UV-Anlage (1) eine Fluidzulaufleitung (12) und eine Fluidauslaufleitung (13) aufweist, gekennzeichnet durch eine erste Desinfektionsmittel-Zufuhreinrichtung (9,11,19), die eine in die Fluidzufuhrleitung (12) mündende erste Desinfektionsmittel-Zufuhrleitung (19) aufweist, eine zweite Behandlungsmittel-Zufuhreinrichtung (10,11,20), die eine in die Fluidauslassleitung (13) mündende zweite Behandlungsmittel-Zufuhrleitung (20) aufweist, durch eine $CO_2$-Zufuhreinrichtung (21-26) mit einer $CO_2$-Zufuhrleitung (22), die in einen an einer Stelle zwischen der ersten Desinfektionsmittel-Zufuhrleitung (19) und der UV-Anlage (1) in einen in der Fluidzulaufleitung (12) angeordneten Mischreaktor (23) mündet, und durch eine Sensorenanlage (27 bzw. 27a), die dazu dient, den Zustand des Fluides in der Fluidzulaufleitung (12) oder der Fluidauslaufleitung (13) abzutasten.

2. Vorrichtung nach Anspruch 1, gekennzeichnet durch eine erste Steuereinrichtung (28), die signaleinganseitig mit der Sensorenanlage (27 bzw. 27a) und signalausgangseitig mit der UV-Anlage (1) und den Desinfektions- bzw. Behandlungsmittel-Zufuhreinrichtungen (9,11,19; 10,11,20) verbunden sind.

3. Vorrichtung nach Anspruch 1 oder 2, gekennzeichnet durch eine zweite Steuereinrichtung (29), die signaleingangseitig mit der Intensitätsmessonde (3) und signalausgangseitig mit mindestens einer der Desinfektions- bzw. Behandlungsmittel-Zufuhreinrichtungen (9,11,19; 10,11,20), der UV-Intensitätssteuereinheit (7), der mechanischen UV-Lampen-Reinigungseinrichtung (4,5,6) und einem Alarmgeber (31) verbunden sind.

4. Vorrichtung nach einem der vorangehenden Ansprüche, gekennzeichnet durch eine von der Fluidauslassleitung (13) abzweigende Spülleitung (18) mit einem ersten Magnetventil (17), durch ein in Durchlaufrichtung des zu entkeimenden Fluides in der Fluidauslassleitung (13) nach der Abzweigstelle der Spülleitung (18) angeordnetes zweites Magnetventil (16), und durch ein in der $CO_2$-Zufuhrleitung (22) angeordnetes drittes Magnetventil (25), welche Magnetventile (17,16,25) steuerseitig mit-

einander in Verbindung stehen.

Fig. 1

Fig. 2

EP 0 811 578 A2